# EUROPEAN PATENT APPLICATION

(11) **EP 1 930 728 A1**
(43) Date of publication of application: **11.06.2008**
(21) Application number: 03712142.3
(22) Date of filing: 14.03.2003
(51) Int. Cl.: G01N 33/68, G01N 33/574, G01N 33/577, C07K 14/705, C07K 16/28

(54) **USE OF THE MAL PROTEIN AS A TUMOUR MARKER**

(30) Priority: 14.03.2002 ES 200200615
(71) Applicant: CONSEJO SUPERIOR DE INVESTIGACIONES CIENTIFICAS, 28006 Madrid (ES)
(72) Inventor: ALONSO LEBRERO, Miguel A., Ctro. "Severo Ochoa", 28049 Madrid (ES); MARAZUELA AZPIROZ, Monica, Hos. Univ. Princesa, 28006 Madrid (ES); ACEVEDO BARBER, Agustin L., Hos Univ. Princesa, 28006 Madrid (ES)
(74) Representative: Carpintero Lopez, Francisco
(86) International application number: PCT/ES2003/000119
(87) International publication number: WO 2003/076948

(57) **Abstract**

The invention relates to the use of the MAL protein as a novel marker and molecular target in epithelial tumours and other human pathologies. More specifically, the invention relates to the use of the detection of the MAL protein as a marker for diagnosis or prognosis purposes for tumours or other pathologies that affect epithelial cells, T lymphocytes, mast cells, myelin-forming cells or other cell types which normally express the MAL protein.

## Description

### TECHNICAL FIELD

The present invention deals with the use of the MAL protein as a novel marker and molecular target in epithelial tumours and other human pathologies. The primary field where this invention is comprised shall be the pharmaceutical/biodiagnosis sector and the field of application of this invention shall be the sanitary/clinical sector.

### STATE OF THE ART

The characterization for diagnosis and/or prognosis purposes of the different types of tumours is realized by the morphological examination of the tumour, the histological analysis of the tumour cells, the inmunohistochemical analysis of the expression of markers associated to the tumour progression, and, in some cases, these valuations are also supported by genetic, cytogenetic data or are supported by any other nature data. The inmunohistochemical analysis of the expression or lack of expression of specific markers is one of the methods that allows the detailed characterization of the neoplasia stage and the establishment of guideline treatment (Rosai and Ackermann, 1996)

The MAL human gene was initially identified during the searching for genes expressed in the differentiation process of T lymphocytes (Alonso and Weissman, 1987) and was mapped on the short arm of the human chromosome 2 (Alonso et al., 1988). Besides expressing in T lymphocytes, the MAL gene expresses moreover in myelin-forming cells (oligodendrocytes and Schwann cells) (Kim et al., 1995; Schaeren-Wiemers et al., 1995) and in some polarized cell lines of epithelial origin (Zacheti et al., 1995; Martín-Belmonte et al., 1998). The protein codified by the MAL gene, simply called MAL, is an integral membrane protein of 17 kilodaltons approximately, with several hydrophobic segments and some potentially immunogenic hydrophobic regions (Alonso and Weissman 1987; Weissman and Alonso 1989). The generation of a mouse monoclonal antibody, called monoclonal antibody 6D9, that recognizes the human protein MAL, gave way to the biochemical and functional characterization of MAL (Martin-Belmonte et al., 1998; Millán and Alonso, 1998). The MAL protein can be purified if associated to a portion of cell membraces presenting a high content in glycolipids and cholesterol. Said portion is composed of a complex mix of lipid membranes whose main feature is to be insoluble in nonionic detergents (such as Triton X-100) at 4°C (Brown and Rose, 1992). In the intact cell, these membranes form microdomains named as well "lipid rafts", that are distributed in the surface or in the inner cell (Simons and Ikonen, 1997; Brown and London, 2000). The existence of the lipid rafts was initially proposed to explain the transport of certain proteins to the apical surface in polarized epithelial cells (Simons and Wanding-Ness, 1990), and more recently these lipid rafts have been involved also in the intracellular protein transport in other cellular types and in cell signaling processes in T lymphocytes, B lymphocytes, mast cells and fibroblasts (Simons and Ikonen, 1997; Alonso and Millán,2001). In polarized epithelial cells, and also in T lymphocytes, MAL spreads mainly in intracellular structures and in lesser proportion in the plasma membrane (Millán et al., 1997). A series of researchs using antisense oligonucleotides to reduce levels of MAL protein using as models epithelial polarized cellular lines called Madine-Darby canine kidney (MDCK) and Fischer rat tyroid (FRT) cells, have shown that the MAL protein is an essential component of the protein machinery implied in the transport to the apical surface of integral membrane proteins as well as glycosylphosphatidylinositol-anchored proteins and secreted proteins (Puertollano et al., 1999; Cheong et al., 1999; Martin-Belmonte et al., 2000, 2001).

In T lymphocytes, in addition to having possibly a function in the intracellular transport (Millán et al., 2002), the MAL protein could be involved in cell signaling processes as suggests his association with the LCK protein tyrosine kinase, an essential component of the activation machinery in these cells (Millán and Alonso, 1998; Alonso and Millán, 2001).

The transport of proteins to the plasma membrane is an essential process in the normal functioning of every type of cell. The loss of cellular polarity is an extremely common phenomenon in the neoplasic transformation and other pathologies (Schoenenberger and Matlin, 1991; Fish and Molitoris, 1994) and most probably one of the causes of these abovementioned pathologies. Neither is known in detail how the tumoral transformation could affect the transport machinery components nor how the maintenance or loss of expression of some of its components could affect the later development of the tumour or its treatment. All these considerations have led us to examine the expression of the MAL protein in different epithelia and other cellular types and investigate if there are differences of subcellular expression and/or distribution of the MAL protein when his expression is compared in normal tissue and different types of tumours coming from the same tissues. Despite its important function in the transport process machinery regarding epithelial cells, the MAL protein has not been so far used as a marker for the characterization of tumours or other pathologies affecting epithelial cells. Neither has been used as a marker in the case of pathologies where the affected cells were T lymphocytes, mast cells, myelin-forming cells or any other cellular types that commonly express the MAL, except in the case of the primary B-cell lymphoma of the mediastinum,where a good correlation with the MAL expression has been shown (Copie-Bergman et al., 1999).There is no certainty if the expression of MAL in this last case is caused by anomalous expression in these cells, or on the contrary, indicates a minor population of B lymphocytes, that could be the forerunners of the mediastinal B-cell lymphoma.

### BIBLIOGRAPHY

1. Alonso M. A., and Millán J. (2001). The role of lipid rafts in signalling and membrane trafficking in T lymphocytes. J. Cell Sci. 114: 3957-3965.
2. Alonso, M. A.; Weissman, S. M. (1987). cDNA cloning and sequence of MAL, a hydrophobic protein associated with human T-cell differentiation. Proc. Nat. Acad. Sci. USA 84: 1997-2001.
3. Alonso M.A., Barton D.E., Francke U. (1988). Assignment of the T-cell differentiation gene MAL to human chromosome 2, region cen-q13. Immunogenetics 27: 91-95.
4. Brown, DA, and London, E. (2000) Structure and function of sphingolipid-and cholesterol-rich membrane rafts. J. Biol. Chem. 275: 17221-17224.
5. Brown, D.A. and Rose, J.K. (1992) Sorting of GPI-anchored proteins to glycolipid-enriched membrane subdomains during transport to the apical cell surface. Cell 68: 533-544.
6. Cheong K.H., Zacchetti D., Schneeberger E.E., Simons K. (1999) VIP17/MAL, a lipid raft-associated protein, is involved in apical transport in MDCK cells. Proc.Natl.Acad.Sci.USA 96:6241-6248.
7. Copie-Bergman C, Gaulard P, Maouche-Chretien L, Briere J, Haioun C, Alonso MA, Romeo PH, Leroy K. (1999). The MAL gene is expressed in primary mediastinal large B-cell lymphoma.Blood. 94:3567-3575.
8. Fish E.M., Molitoris B.A.. (1994). Alterations in epithelial polarity and the pathogenesis of disease states. New Engl. J Med. 330:1580-1588.
9. Kim, T., Fiedler, K., Madison, D.L., Krueger, W.H., and Pfeiffer, S.E. (1996). Cloning and characterization of MVP17: a developmentally regulated myelin protein in oligodendrocytes. J. Neurosci. Res. 42:413-422.
10. Molecular Cloning: A Laboratory Manual (Cold Spring Manual Laboratory 1989)
11. Marazuela, M., Sánchez-Madrid, F., Acevedo, A., Larrañaga, E. and Landazuri, M.O. (1995) Expression of vascular adhesion molecules on human endothelia in autoimmune thyroid disorders. Clin. Exp. Immunol. 102: 328-334.
12. Martin-Belmonte F, Arvan P, Alonso MA. (2001) MAL mediates apical transport of secretory proteins in polarized epithelial Madin-Darby Canine Kidney cells. J. Biol. Chem.; 276: 49337-49342.
13. Martin-Belmonte F, Kremer L, Albar JP, Marzuela M, Alonso M. (1998) Expression of the MAL gene in the thyroid: The MAL proteolipid, a component of glycolipid-enriched membranes, is apically distributed in thyroid follicles. Endocrinology 139:2077-2084.
14. Martin-Belmonte F., Puertollano R., , Millan J., and Alonso M.A.(2000) The MAL proteolipid is necessary for the overall apical delivery of membrane proteins in the polarized epithelial Madin-Darby canine kidney and Fischer rat thyroid cell lines. Mol. Biol. Cell 11:2033-2045.
15. Millán, J. and Alonso, M. A. (1998). MAL, a novel integral membrane protein of human T lymphocytes, associates with glycosylphosphatidylinositol-anchored proteins and Src-like tyrosine kinases. Eur. J. Immunol. 28, 3675-3684.
16. Millán J., Montoya M. C., Sancho D., Sánchez-Madrid F. and Alonso M. A. (2002). Lipid rafts mediate biosynthetic transport to the T lymphocyte uropod subdomain and are necessary for uropod integrity and function. Blood 99:978-984.
17. Millan J., Puertollano R., Fan Li., Rancano C., Alonso M.A. (1997) The Mal proteolipid is a component of the detergent-insoluble membrane subdomains of human T-lymphocytes. Biochem J 321:247-252
18. Murphy W. M., Beckwith J.B. and Farrow G.M. (1994) Tumors of The kidney, bladder and related urinary structures. In Atlas of tumor pathology. Rosai J., and Sobin L.H. (Eds) Published by the Armed Forces Institute of Pathology. Bethesda p.92-136
19. Puertollano R., Martin-Belmonte F., Millán J., de Marco M.C., Albar J. P., Kremer L., and Alonso M. A. (1999). The MAL Proteolipid Is Necessary for Normal Apical Transport and Accurate Sorting of the Influenza Virus Hemagglutinin in Madin-Darby Canine Kidney Cells. J. Cell Biol., 145: 141-145.
20. Razani B., Schlegel A., Lisanti M.P. (2000). Caveolin proteins in signaling, oncogenic transformation and muscular dystrophy, J Cell Sci 113:2103-2109.
21. Rosai J., Ackerman S. (1996) Surgical Pathology. Mosby Inc., St. Louis, Mo.
22. Rosai J., Carcangui ML and Delellis RA (1992) Tumours of the Thyroid Gland. In Atlas of tumor pathology. Rosai J., and Sobin L.H. (Eds) Published by the Armed Forces Institute of Pathology. Bethesda, p.19-161.
23. Sambrook J., Fritsch E.F., and Maniatis T. (1989). Molecular Cloning. A Laboratory Manual. Cold Springs Harbor Laboratory Press. NY.
24. Schaeren-Wiemers N., Valenzuela D.M., Frank M., Schwab M.E. (1995). Characterization of a rat gene, rMAL, encoding a protein with four hydrophobic domains in central and peripheral myelin. J Neurosci. 15:247-252.
25. Schoenenberger C.A.and Matlin K.S. (1991). Cell polarity and epithelial oncogenesis. Trends Cell Biol. 1:87-92.
26. Simons K., Ikonen E. (1997). Functional rafts in cell membranes. Nature 387:569-572.
27. Simons, K. and Wandinger-Ness, A (1990). Polarized sorting in epithelia. Cell 62:207-210.
28. Weissman S.M., Alonso M.A. (1989). T-cell membrane protein. Patent Number: 4.835.255. Date of Patent: May 30, 1989. Owner of Patent: Yale University. New Haven. Conn. Country of Patent: U.S.A.
29. Zacchetti D., Peranen J., Murata M., Fiedler K., Simons K. (1995). VIP17/MAL, a proteolipid in apical transport vesicles. FEBS Lett 377:465-469.

### DESCRIPTION OF THE INVENTION

### Brief description of the invention

The integral membrane protein called MAL has been characterized as an essential component of the machinery for polarized transport in epithelial cells (Puertollano et al., 1999, Martín-Belmonte et al., 2000 and 2001). To characterize the different cell types that express the MAL protein and subsequently correlate the alterations in the expression and/or distribution with the development in pathological cell stages, we have firstly carried out an extensive immunohistochemical analysis of the MAL expression in a wide range of human tissues using the mouse monoclonal antibody named 6D9 that recognizes the human MAL protein. This analysis has allowed us to identify the MAL expression in specific types of epithelial cells, T lymphocytes, mast cells and myelin-forming cells. In the immunohistochemical study of various tumoral types, taken as pathology examples affecting these tumoral types, we have observed that some particular types of tumours do not show MAL expression and/or present any alteration in its cellular distribution. This discovery suggests that the study of the expression and/or distribution of MAL in tumours and other pathological tissues can be useful for a better characterization of the pathological samples for either prognostic or diagnostic purposes, for detection of incipient neoplasias and a better knowledge of the tumoral transformation. Particularly, the present invention deals with the use of detection of MAL protein as a marker for either prognostic or diagnostic purposes in tumours or other pathologies that affect epithelial cells, T lymphocytes, mast cells and myelin-forming cells or other cell types that commonly express MAL protein (excluding the primary B-cell lymphoma of the mediastinum). Another specific aim of the present invention is the use of Mal protein detection by means of antibodies, either monoclonal or polyclonal, that specifically recognize the MAL protein. The processes and methods in the preparation and development of monoclonal or polyclonal antibodies that specifically recognize MAL protein are widely described and well known in the state of the art (Martin-Belmonte et al., 1998; Millán et al., 1998), therefore, the use of these antibodies in the detection of the MAL protein in tumour cells is part of the present invention. A more specific object of the present invention is the use of the monoclonal antibody anti-MAL 6D9 in detection and identification procedures of MAL protein in normal and tumour cells, among other possibilities, by means of immunohistochemistry. On the other hand, the identification of the sequence of nucleotides codifying MAL protein, for example RNA, and whose expression gives as a result that said protein can be developed by existing processes and tools in the state of the art (Sambrook et al., 1989), among others, the Northern blot analysis using specific and complementary probes or the polymerase chain reaction using template cDNA (RT-PCR) are part of the present invention.

The loss of cellular polarity is an extremely common phenomenon in neoplasic transformation and other pathologies, modifies the morphology and characteristics of the affected cells (Schoenenberger and Matlin, 1991; Fish and Molitoris, 1994) and it is related to its etiopathogeny as it has been demonstrated in the case of the proteins called caveolins, which are also components in the transport machinery (Razani et al., 2000). Therefore treatments that modify the levels of MAL protein, which is an essential component of the transport machinery in the pathological cells, might dramatically alter the transport in these cells, as occurs in the normal polarized cells (Puertollano et al., 1999, Martin-Belmonte et al., 2000) and modify therefore the course of the disease or its therapeutic treatment.

### Detailed description of the invention

We have carried out an immunohistochemical study of the expression and distribution of the MAL protein in a wide range of normal human tissues and compared the results with those obtained in the analysis of tumours, as example of pathologies affecting these tissues. We have observed differences in the levels of expression and/or the distribution pattern of MAL among normal tissue and some of the tumours analysed.

### 1. Results of MAL expression obtained in normal human tissues

The MAL protein expression in different epithelia as well as in endocrine and exocrine glands, and the absence of detectable levels of MAL expression in fibroblasts, endothelial cells and skeletal and non-striated muscle are some of the common features observed in the immunohistochemical analysis we have carried out in normal human samples whose result has been summarized in Table I. Other general observations found in the course of the analysis are the existence of MAL expression in mast cells, and in the myelin surrounding the axons in the peripheral nerves (Table I)

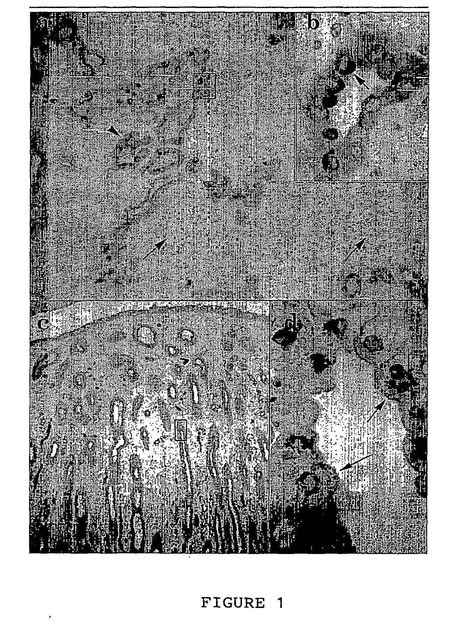

A brief summary of some of the outstanding results obtained in the immunohistochemical analysis of MAL expression in normal human samples are described bellow:
- In the skin, MAL expression was found in the apical region of the eccrine cells of the sweat glands and in the acrosyringium but it was neither found in the keratinized flat epithelium, nor in the dermis or in the subcutaneous fibrofatty tissue.
- In the evaluation of the MAL expression in the gastrointestinal tract, sample tissues of esophagus, stomach, ileum, colon, liver and pancreas were analysed. The stratified flat epithelium was positive to the MAL expression, as the stain was more intense in the most apical cell layers. When the stratified epithelium ends in the gastric epithelium, the flat cylindrical epithelium of the stomach, which includes mucus producer cells, showed positive stain in the MAL expression. Moreover, specially in parietal cells, which produce acid and intrinsic factor, and also the principal cells, that secrete digestive enzymes, were highly immunoreactive with the anti-MAL antibody. In the small intestine, the base cells of Lieberkuhn's crypts, which include de Paneth cells, were positive with a stain that was gradually decreasing as it went towards the villus surface. The cells in the villi, enterocytes and mucus producer cells presented a similar MAL distribution pattern as the observed in other polarized epithelia, consisting in a strong supranuclear staining of granular character and undetectable staining in the basal or lateral surfaces. This distribution corresponds to the Golgi apparatus area. In the Peyer plaques, the lymphocytes in the paracortical region were positive as well as some sporadic cells in the germinal center. The endothelial cells that form the high endothelial venules were positive with the common MAL supranuclear distribution. Through all the gastrointestinal tract, the myenteric plexus and the submucosal plexus were strongly positive to the MAL expression. On the contrary, the flat muscle of the muscle sheath of the mucous membrane and the striated muscle of the muscle sheath did nor show staining with the antibody anti-MAL. The expression and distribution of MAL in the large intestine were similar to those above described in the small intestine.
- In normal liver, the hepatocytes located around the centrilobular area showed granular cytoplasmic staining with the antibody anti-MAL.The sinusoidal surfaces and the Kupffer cells were negative. The bile duct epithelium was weakly positive. The acinic cells of the exocrine pancreas showed granular supranuclear staining. In the endocrine pancreas, some sporadic cells of the islets of Langerhans were positive in the MAL expression.
- Many areas in the genitourinary tract were positive in the MAL expression. Therefore, in the kidney there was found MAL expression in specific regions of the renal tubuli (Figure 1). In the kidney cortex, MAL was detected in the convoluted proximal tubules but not in the distal tubules (Figure 1a). The stain is more pronounced in some cells than in others (Figure 1b). The glomeruli were negative. In the bone marrow it was observed a strong expression of MAL, with its characteristic supranuclear granule distribution in the collecting tubules (Figure 1c and d). The transitional epithelium of the urothelium (bladder and urethra) showed to be highly positive to the MAL expression.
- The hematopoietic tissues analysed included the thymus, the lymph nodes and the tonsil. In the thymus, the MAL expression restricts mainly to the cortex, being the stain in the marrow very inferior. In the lymph nodes and the tonsil, the staining pattern was very similar. The staining was confined to the paracortical lymphocytes (T-cells area) and sporadic lymphocytes inside the follicle. In contrast to the absence of MAL expression in other endothelial cells, in the endothelium of high endothelial venules (postcapillary venules) stained positive to MAL with the common apical distribution. Dendritic cells and follicular dendritic cells were negative.
- In the lung, the ciliated cylindrical epithelium and the mucus producer cells of bronchi and bronchioli showed positive staining. The staining was confined to the cells's apical part. In the acini, type 1 pneumocytes, which delimit the alveolar walls were negative while type 2 pneumocytes, which secrete surfactant components, were highly positive.
- In the adrenal glands, the marrow presented an intense labeling for MAL expression with typical granular distribution. Although all the cortex layers were positive, MAL expression was more pronounced in the reticular area. As we have previously described (Martin-Belmonte et al, 1998), the thyroid epithelium was stained for MAL with its characteristic granular supranuclear distribution. In testis, Leydig cells were highly stained with the antibody, while Sertoli cells showed a weaker staining.The epithelium of the prostate glands also showed to be positive for MAL expression.

**Table I. Summary of results obtained about MAL distribution in different tissues.**

| Tissue/Organ | High expression | Weak or focalized expression | Absence of detectable expression |
|---|---|---|---|
| Common structures | Axons Mast cells | | Endothelial cells Fibroblasts Muscle cells |
| Skin | Eccrine ductal cells | | Keratinized epithelium Keratinocytes Hair follicles Melanocytes |
| Esophagus | Stratified flat epithelium | | Muscle cells Submucosa |
| Stomach | Parietal cells Principal cells | Superficial mucous cells | Muscle cells Submucosa |
| Small intestine | Crypt cells Paneth cells T Lymphocytes (Peyer plaques) | Enterocytes with microvilli | Muscle cells Submucosa |
| Large intestine | Mucous cells | | Muscle cells Submucosa |
| Liver | Centrilobular hepatocytes | Other hepatocytes Intrahepatic ductal epithelium | Kupffer cells Endothelium |
| Pancreas | Acinar cells | Ductal cells Endocrine cells | |
| Kidney | Convoluted proximal tubules Collecting tubules | Henle's loop | Glomeruli Convoluted distal tubules Bowman's capsule Juxtaglomerular apparatus |
| Urethra Bladder | | Superficial cells of urothelium | Other epithelial cells Muscle cells |
| Prostate | Acinar and ductal cells | | |
| Lymph gland | T cells High endothelial venules | | B cells Sinusoidal cells Dendritic cells Macrophages Other endothelia |
| Tonsil | T cells High endothelial venules Superficial and crypt epithelium | | B cells Sinusoidal Cells Macrophages Dendritic cells |
| Thymus | Cortical thymocytes | Medullary thymocytes Hassall's bodies | Epithelial cells Dendritic cells |
| Bronqui and trachea | Mucous cells | Respiratory epithelium | |
| Lung | Type II Pneumocytes | Mucous cells | Type I Pneumocytes |
| Thyroid gland | | Tirocytes | Parafollicular cells |
| Testis | Leydig cells | Sertoli cells | Germinal cells |
| Adrenal glands | Medullary cells Reticular zone | Glomerular zone Trabeculated zone | |

### 2. Results of MAL expression obtained in human tumours

MAL expression and distribution was examined according to immunohistochemical methods in different types of renal and thyroid tumours to be compared with normal tissue. The studied renal tumours included the main varieties of renal carcinoma: clear cell renal carcinoma (5 cases), papillary renal cell carcinoma (5 cases), cromophobic renal cell carcinoma (5 cases), sarcomatoid renal cell carcinoma (2 cases), and renal oncocytoma (3 cases). The studied thyroid tumours included the thyroid papillary carcinoma (4 cases), the follicular carcinoma (2 cases), and anaplasic carcinoma (1 case). The tumour samples were defined according to morphological criteria using sections stained with hematoxiline following the established criteria for the classification of kidney tumours (Murphy et al., 1992) and thyroid tumours (Rosai et al., 1992).

### Renal tumours

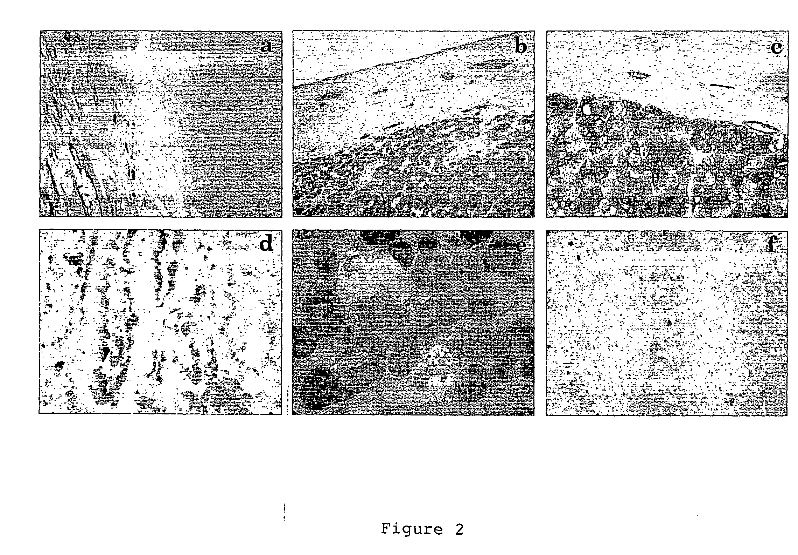

Clear cell renal carcinomas constitute 70 - 80% of renal cell cancer. Histologically, they can be differentiated in two types due to the clear or granular aspect of the cytoplasm. MAL expression was not detected in any of the clear cell renal carcinomas (Fig. 2a). On the contrary, the granular type tumours showed an intense cytoplasmic staining for MAL protein with granular pattern (Fig. 2b). Cromophobic carcinoma constitutes approximately 5% of renal cell carcinomas. The cells of the cromophobic carcinomas showed an intense staining with diffuse distribution, being more intense in the apical part and in some cells more than in others (Fig. 2c). Papillary carcinoma constitutes 10 - 15% of renal carcinomas. The staining in these latter carcinomas was similar to the normal kidney (Fig. 2d). Among benign renal tumours, the oncocytomas constitute approximately 5% of renal neoplasias. The renal oncocytomas examined showed an intense cytoplasmic staining for MAL (Fig. 2e). Sarcomatoid carcinomas constitute approximately 1 % of tumours of renal cells. MAL expression in sarcomatoid renal carcinoma was undetectable (Fig. 2f).

### Thyroid tumours

Papillary carcinoma constitutes approximately 65 - 80% of thyroid tumours.In all the cases of papillary renal cell carcinoma they did not showed staining with the antibody anti-MAL. The follicular thyroid carcinoma, that constitutes 10 - 15% of thyroid tumours, showed a diffuse staining in the apical region in contrast to the granular staining found in the normal thyroid. In anaplasic carcinoma, which is a rare and aggresive tumour that constitutes less than 5% of thyroid carcinomas, only become stained some cells that presented a different membrane pattern than the typical granular pattern of the normal thyroid follicles.

In conclusion, our results show that: 1) MAL protein expresses in a wide variety of cell types, 2) in some types of tumours dramatical changes are produced in the expression of MAL protein, and 3) the observed changes of MAL expression in some types of tumours might be necessary for the malign progression and might determine its morphology and characteristics. Therefore MAL protein can be used as a marker for a more detailed characterization of tumours where MAL usually expresses (items 1 and 2) and the clinical manipulation of the levels of MAL expression could be of use to alter the course of tumoral development or its therapeutic treatment (item 3). Although our research has been centered in the analysis of epithelial tumours, our observations could be widen to other non-identified cell types in the present research and other different types of pathologies of the tumour transformation.

### DESCRIPTION OF FIGURES

**Figure 1****. Immunohistochemical analysis of the expression and distribution of MAL protein in human kidney.** (a) Renal cortex. The convoluted proximal tubules (arrowhead) were positive for MAL expression while the glomerules (arrows) were negative. (b) At higher scale, one can appreciate the differentiate staining pattern observed in the proximal tubules with a more accentuated staining in some cells than in others. (c) Bone marrow. The collecting tubules were very intensely stained with the antibody anti-MAL. (d) At higher scale, one can appreciate in the collecting tubules the typical granular supranuclear distribution of the MAL protein(arrows).
**Figure 2****. Immunohistochemical analysis of the expression and distribution of MAL protein in different types of human renal tumours.** (a) Clear cell renal carcinoma. It was not found expression in the tumoral cells but it stained positive in the normal adjacent cells. (b) Granular carcinoma. Intense staining was found in all the cells. (c) Cromophobic renal cell carcinoma. A diffuse staining was found in all the cells whose intensity varied from some cells to others. (d) Papillary renal cell carcinoma. Staining is more pronounced in the apical side and its intensity varies from some cells to others. (e) Renal oncocytoma. Epithelial cells show an intense granular cytoplasmic staining. (f) Sarcomatoid renal cell carcinoma. There was not found reactivity with the antibody anti-MAL.

### EXAMPLES

### Example 1. Preparation, staining and identification of normal and tumoral tissues with the monoclonal antibody anti-MAL 6D9.

The tissue samples used were received in the Department of Pathology of the Hospital de la Princesa (Madrid) as ordinary specimens obtained by surgery. All samples were fixed with 10% neutral buffered formalin and tested in ordinary processing and paraffin-embedded. 5- µM sections were prepared from the paraffin blocks and mounted on poli-L-lysine coated slides.The sections, once eliminated the paraffin, were subject to a pressure cooker treatment for 1 minute in buffer citrate 200 mM, P.H. 6.0. The samples were blocked aferwards with a dilution 1:20 of normal rabbit serum in saline buffer Tris-HCI P.H. 7.6, as it has been previously described (Marazuela et al., 1995). Sections were sequentially incubated with a diluted preparation 1:50 of monoclonal antibody anti-MAL 6D9 of ascites fluid obtained from mice inoculated with the producer hybridoma of the monoclonal antibody (Martin-Belmonte et al., 1998; Millán et al., 1998) and, afterwards, with rabbit antibodies coupled to peroxidase specific against Rabbit IgG (Dako A/S, Glostrup, Dinamarca). Each incubation was followed with washing (3 times) in saline buffer Tris-HCI. Sections were developed by Graham-Kamovsky medium containing 3,3' -diaminobenzidine tetrachloride (Sigma Chem. Co., St. Louis. USA) and hydrogen peroxide. Sections were also stained with hematoxilin (Carazzi method), dehydrated and mounted according to ordinary methods. At least, 4 samples of each specimen were labelled with a secret code and independently analysed by two specialists.

## Claims

1. Use of detection of MAL protein or genetic precursors as a marker with diagnostic or pronostic purposes for tumours or other pathologies affecting epithelial cells, T Lymphocytes, mast cells, myelin-forming cells or other cell types that usually express MAL protein (excluding primary B-cell lymphomas of the mediastinum).

2. Use of detection of MAL protein according to Claim 1 **characterized** because these are renal and thyroid tumours, among others.

3. Use of detection of MAL protein according to Claims 1 and 2, **characterized** because it is carried out by means of monoclonal or polyclonal antibodies, which specifically recognize MAL protein.

4. Use of detection of MAL protein according to Claim 3, **characterized** because the antibody is the monoclonal antibody anti-MAL 6D9.

5. Use of detection of MAL protein precursors, **characterized** because it is based in the detection of the nucleotide sequences that codify MAL protein, among others, Northern Blot.

6. Use of the manipulation of MAL protein expression in tumoral cells **characterized** because it allows to modify the course of the tumour progression and its therapeutic treatment.
